# EUROPÄISCHE PATENTSCHRIFT

(11) **EP 2 245 981 B1**
(45) Veröffentlichungstag und Bekanntmachung des Hinweises auf die Patenterteilung: **19.09.2018**
(21) Anmeldenummer: 10004463.5
(22) Anmeldetag: 28.04.2010
(51) Int. Cl.: A61B 90/00

(54) **Fernbedienung für ein Operations-Assistenz-System**
Remote control for an operations assistance system
Télécommande pour un système d'assistance à l'opération

(30) Priorität: 28.04.2009 DE 102009018918
(43) Veröffentlichungstag der Anmeldung: 03.11.2010
(73) Patentinhaber: Aktormed GmbH, 93092 Barbing (DE)
(72) Erfinder: Geiger, Robert, Dipl.-Ing. (FH), 94526 Metten (DE); Scherr, Jürgen, 93464 Tiefenbach (DE); Kraus, Peter, 93059 Regensburg (DE)
(74) Vertreter: Glück Kritzenberger Patentanwälte PartGmbB

(56) Entgegenhaltungen:
- WO-A1-99/08611
- DE-A1-102004 041 871
- US-A1- 2001 034 530
- US-A1- 2008 188 870

## Beschreibung

Die Erfindung bezieht sich auf eine Fernbedienung für ein Operations-Assistenz-System gemäß dem Oberbegriff des Patentanspruches 1.

Drahtlose Fernbedienungen zur Steuerung von entfernt angeordneten elektronischen und/oder elektromechanischen Systemen und/oder Geräten sind hinlänglich bekannt. Diese bestehen beispielsweise aus einem mehrteiligen Gehäuse und zumindest einem Bedienelement, wobei das mehrteilige Gehäuse zumindest ein unteres Gehäuseteil zur zumindest teilweisen Aufnahme einer Elektronikeinheit und ein oberes Gehäuseteil, in dem vorzugsweise das zumindest einem Bedienelement vorgesehen ist, aufweist.

Insbesondere finden Fernbedienungen im medizinischen Bereich, insbesondere bei minimal invasiven Operationen häufig Verwendung. Diese werden insbesondere zur Steuerung von chirurgischen Hilfsinstrumenten, beispielsweise eines Kamerasystems innerhalb eines Operations-Assistenz-System vorgesehen. Ein derartiges Operations-Assistenz-System ist beispielsweise der DE 103 52 197 zu entnehmen.

Aus der WO 2007/038998 A1 ist beispielsweise eine Fernbedienung zur drahtlosen Fernbedienung eines entfernt angeordneten medizinischen Gerätes bekannt, welches zur Befestigung ein einem chirurgischen Handinstrumentes ausgebildet ist, wobei das chirurgische Handinstrument für minimal invasive Eingriffe vorgesehen ist.

Nachteilig sind bekannte Fernbedienungen für medizinische Geräte oder Systeme als Einwegartikel ausgebildet, d.h. diese werden nach der Herstellung steril verpackt und unmittelbar nach dem Gebrauch, d.h. nach der Operation, entsorgt. Somit stehen diese für weitere Operationen aus sterilisationstechnischen Gründen nicht mehr zur Verfügung.

Insbesondere ist dies dadurch bedingt, dass derartige Fernbedienung nicht autoklavierbar sind, d.h. diese können keinesfalls in einem Dampfbad eines Autoklaven auf ggf. über 136° Celsius erhitzt und somit für eine erneute Verwendung sterilisiert werden ohne das eine Beschädigung der Fernbedienung und/oder Beeinträchtigung deren Funktionsweise vorliegt.

Zudem sind beispielsweise aus den Druckschriften DE 10 2004 041 871 A1, US 2001/034530A1 sowie WO 99/08611A1 Fernbedienungen für Operations-Assistenz-Systeme bekannt geworden, die aus einem mehrteiligen Gehäuse zur Aufnahme zumindest einer Elektronikeinheit und zumindest einem mit der Elektronikeinheit verbundenen Bedienelementes bestehen, wobei das Gehäuse zumindest ein oberes und unteres Gehäuseteil aufweist, die zur Ausbildung eines autoklavierbaren Gehäuses hermetisch dicht miteinander verbunden sind.

Ausgehend vom vorliegenden Stand der Technik ist es Aufgabe der Erfindung, eine gegenüber dem bekannten Stand der Technik weiterentwickelte Fernbedienung für ein Operations-Assistenz-System anzugeben, welche sterilisierbar und somit mehrfach verwendbar ist. Die Aufgabe wird ausgehend von den Merkmalen des Oberbegriffes des Patentanspruches 1 durch dessen kennzeichnende Merkmale gelöst.

Ein wesentlicher Aspekt der erfindungsgemäßen Fernbedienung für ein Operations-Assistenz-System ist darin zu sehen, dass der untere Gehäuseteil zumindest eine Ausnehmung zur Aufnahme einer Batterieeinheit und zumindest eine Batterieauswurfbohrung zum vereinfachten Entnehmen der Batterieeinheit aus der Ausnehmung aufweist, die mit der Ausnehmung in Verbindung steht, und dass die Ausnehmung eine in einem ersten Gehäuserandabschnitt vorgesehene Aufnahmeöffnung und die Batterieauswurfbohrung eine in einem zweiten Gehäuserandabschnitt vorgesehene erste Bohrungsöffnung und im Bodenbereich der Ausnehmung eine zweite Bohrungsöffnung aufweist, wobei die Aufnahmeöffnung und die vorgesehene erste Bohrungsöffnung einander gegenüberliegend angeordnet sind. Besonders vorteilhaft kann das hierdurch entstehende hermetisch abgedichtete, mehrteilige Gehäuse mittels einem Autoklaven in der an sich bekannten Art und Weise sterilisiert werden und somit für weitere vorzugsweise minimal invasive Operationen wieder verwendet werden. Zum vereinfachten Entnehmen der Batterieeinheit aus der Ausnehmung ist in der erfindungsgemäßen Fernbedienung zumindest eine Batterieauswurfbohrung im Gehäuse, und zwar im unteren Gehäuseteil vorgesehen, die mit der Ausnehmung in Verbindung steht. Besonders bevorzugt sind hierzu das obere Gehäuseteil und das untere Gehäuseteil randseitig miteinander verklebt.

Besonders vorteilhaft ist die Ausnehmung vorzugsweise zur näherungsweise formschlüssigen Aufnahme der Batterieeinheit ausgebildet. Die Batterieeinheit stellt hierbei einen Einwegartikel dar, welcher nach erfolgter Operation entsorgt wird. Besonders vorteilhaft ist die Batterieeinheit in die hinsichtlich Form und Dimensionierung an die Batterieeinheit angepasste Ausnehmung im Gehäuse einfach und schnell einführbar, d.h. die ggf. bereits mehrfach benutzte ist nach erneuten Autoklavieren durch Einfachen einer neuen, sterilisierten Batterieeinheit sofort wieder verwendbar. Die Batterieeinheit ist hierbei derart dimensioniert, dass die von dieser bereitgestellte elektrische Energie zur Durchführung einer mehrstündigen Operation problemlos ausreicht.

Mittels eines längen, stiftartigen Gegenstandes, beispielsweise dem Griff eines chirurgischen Handinstrumentes ist somit die Batterieeinheit besonders einfach und schnell aus der Ausnehmung zu entfernen.

Weiterbildungen, Vorteile und Anwendungsmöglichkeiten der erfindungsgemäßen Fernbedienung sind Gegenstand der abhängigen Ansprüche.

Die Erfindung wird im Folgenden anhand der Figuren an einem Ausführungsbeispiel näher erläutert. Es zeigen:
- Fig. 1: eine perspektivische Draufsicht auf ein chirurgischen Instruments für minimal invasive Eingriffe mit einer erfindungsgemäßen Fernbedienung für ein Operations-Assistenz-Systems;
- Fig. 2: eine perspektivische Seitenansicht der am chirurgischen Instrument gemäß Figur 1 vorgesehenen erfindungsgemäßen Fernbedienung;
- Fig. 3: eine perspektivische Ansicht einer Stirnseite der am chirurgischen Instrument gemäß Figur 1 und 2 vorgesehenen erfindungsgemäßen Fernbedienung;
- Fig. 4: eine perspektivische Seitenansicht der erfindungsgemäßen Fernbedienung mit freigeschnittener Ausnehmung ohne Batterieeinheit;
- Fig. 5: eine perspektivische Seitenansicht der erfindungsgemäßen Fernbedienung gemäß Fig. 4 mit Batterieeinheit;
- Fig. 6: einen Längsschnitt durch die erfindungsgemäßen Fernbedienung ohne Batterieeinheit und
- Fig. 7: einen Längsschnitt durch die erfindungsgemäßen Fernbedienung mit in der Ausnehmung aufgenommener Batterieeinheit.

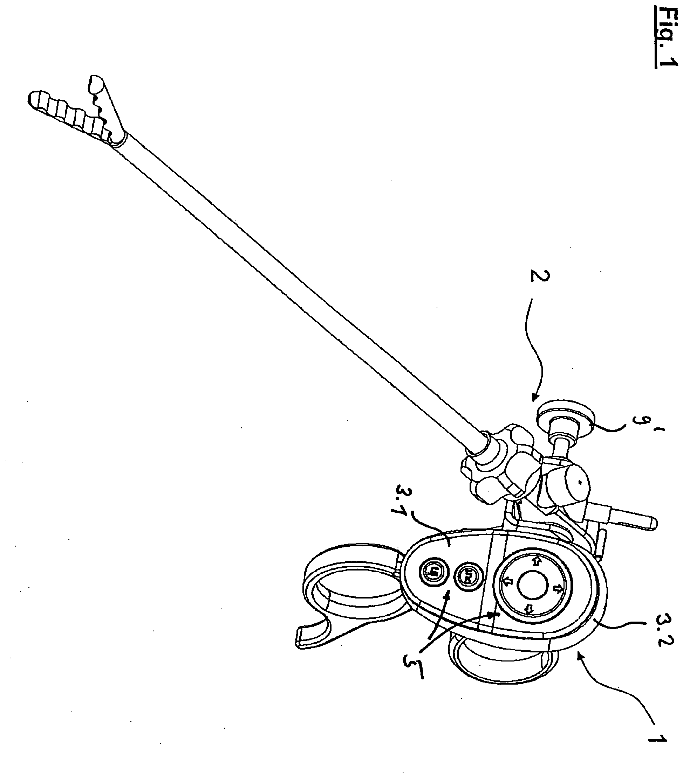

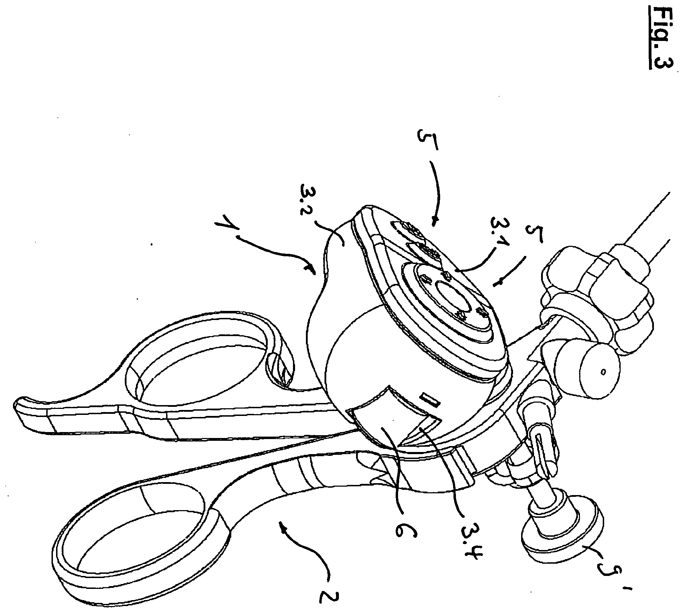

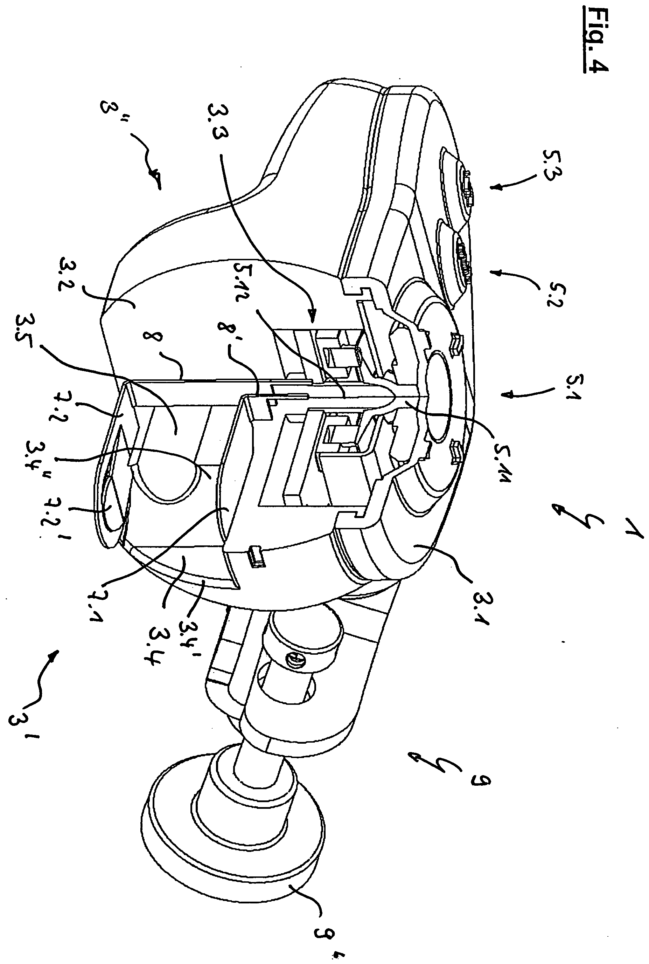

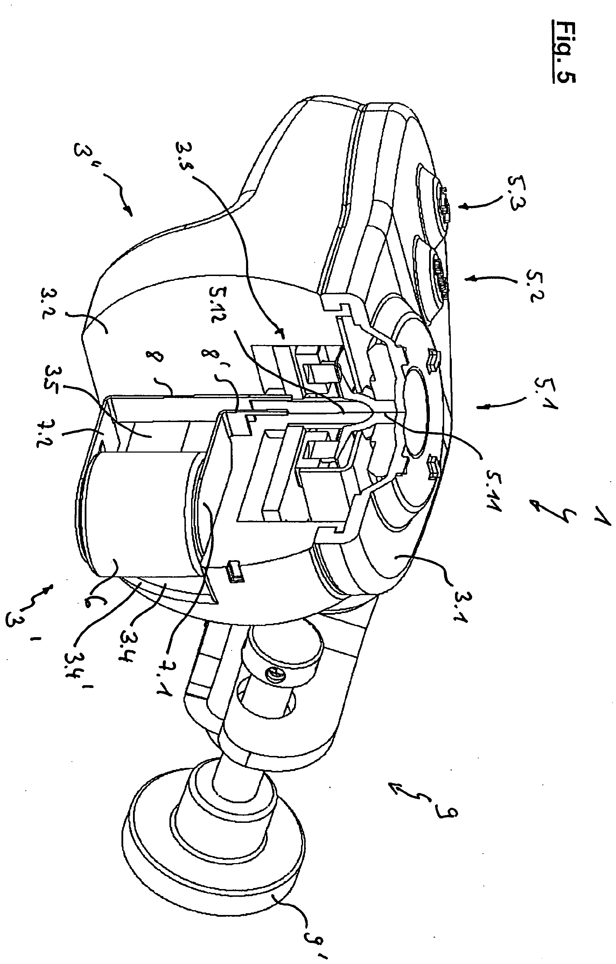

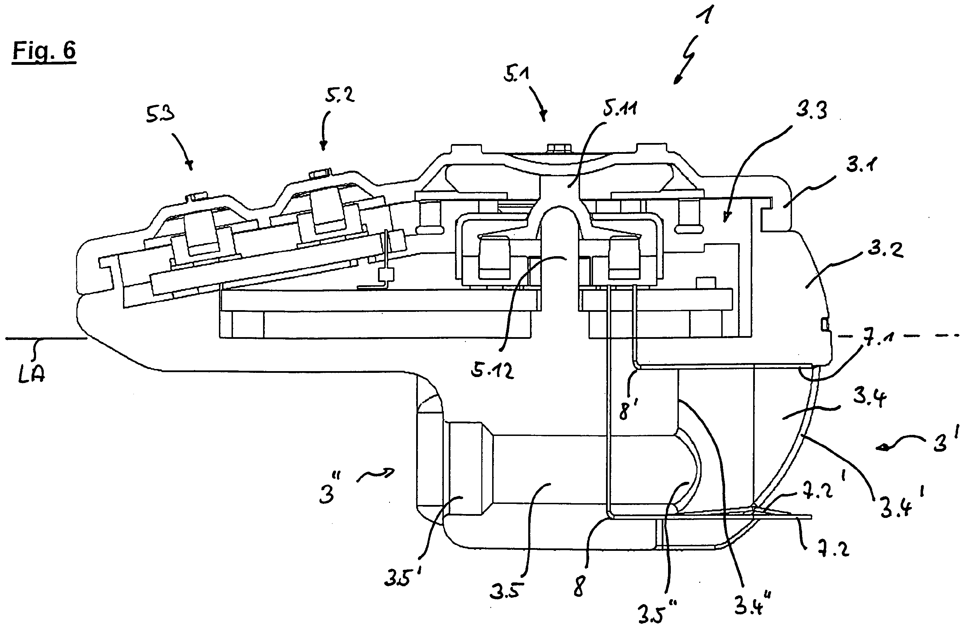

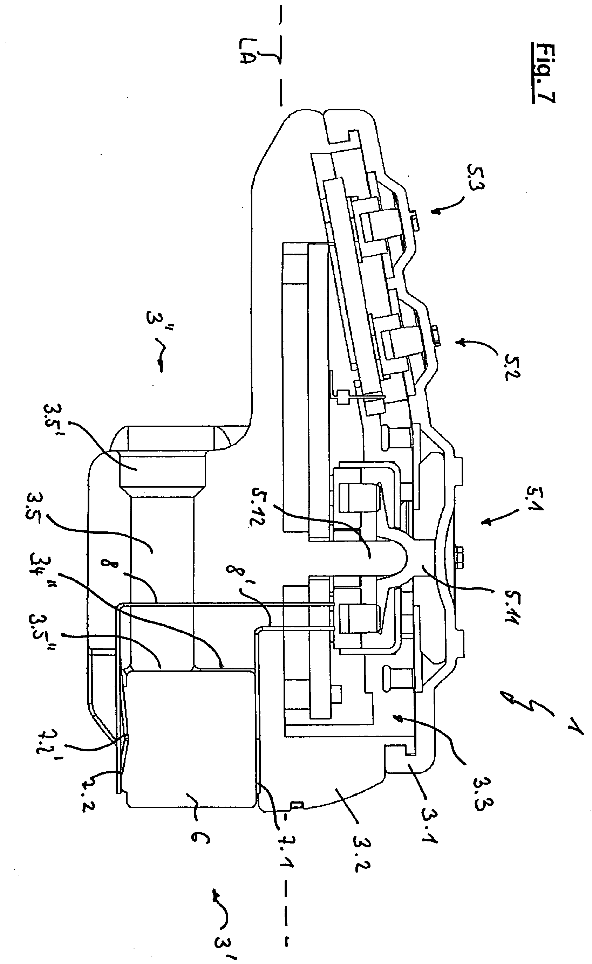

In den Figuren 1 bis 7 ist mit 1 jeweils eine Fernbedienung für ein Operations-Assistenz-System bezeichnet, welches insbesondere zur Steuerung eines chirurgischen Instruments oder Hilfsinstruments, insbesondere ein Endoskop aufweisenden Kamerasystems während einer minimal invasiven Operation vorgesehen ist.

Das chirurgische Instrument bzw. Hilfsinstrument wird hierbei über eine kleinformatige Operationsöffnung ("Trokar") in einen Operationsraum eines Patientenkörpers eingeführt und über das Operations-Assistenz-System gemäß den von der Fernbedienung 1 erzeugten Steuerbefehlen innerhalb des Patientenkörpers gesteuert. Die Fernbedienung 1 ist hierzu beispielsweise als Handbedienteil ausgebildet, welche über eine drahtlose Schnittstelle mit einer Steuereinrichtung des Operations-Assistenz-Systems verbindbar ist.

Die erfindungsgemäße Fernbedienung 1 ist beispielsweise - wie in den Figuren 1 bis 3 dargestellt - zur Befestigung an einem chirurgischen Instrument 2, beispielsweise einer Greiferzange ausgebildet und somit schnell und einfach durch den Operateur während eines minimal invasiven Eingriffes bedienbar.

Die erfindungsgemäße Fernbedienung 1 umfasst ein mehrteiliges Gehäuse 3 zur Aufnahme zumindest einer Elektronikeinheit 4 und zumindest einem mit der Elektronikeinheit 4 verbundenen Bedienelementes 5. Insbesondere weist das mehrteilige Gehäuse 3 zumindest ein oberes Gehäuseteil 3.1 und unteres Gehäuseteil 3.2 auf, die zur Ausbildung eines autoklavierbaren Gehäuses 3 hermetisch dicht miteinander verbunden sind. Hierzu sind das zumindest eine obere und untere Gehäuseteil 3.1, 3.2 randseitig miteinander verklebt, und zwar derart, dass diese einen geschlossenen, hermetisch abgedichteten Aufnahmeraum 3.3 zur Aufnahme der Elektronikeinheit 4 und des zumindest einen Bedienelementes 5 ausbilden. Hierbei weist das untere Gehäuseteil 3.1 eine umlaufende Befestigungsnut auf, in welche eine ebenfalls umlaufende Befestigungsfeder des oberen Gehäuseteils 3.2 eingreift, und zwar vorzugsweise form- und/oder kraftschlüssig.

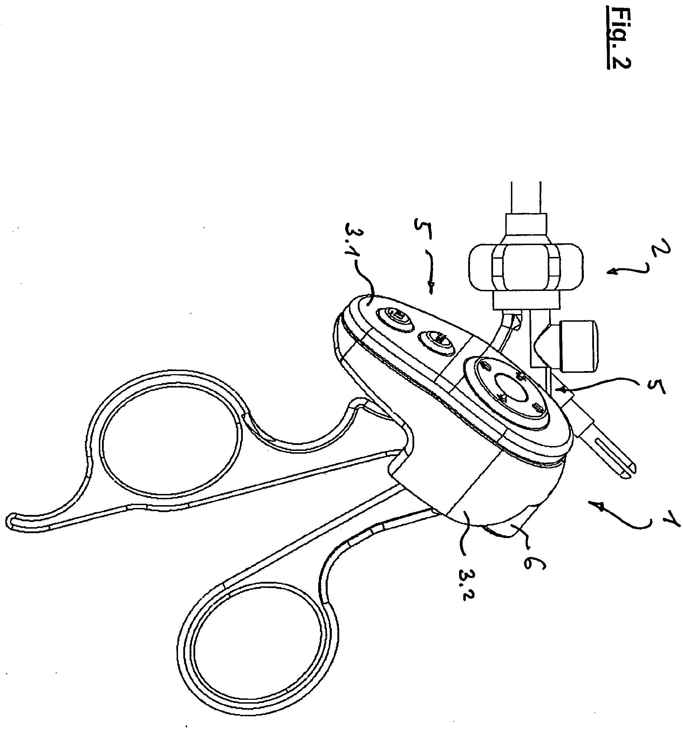

Die Fernbedienung 1 weist ferner zur Versorgung der Elektronikeinheit 4 mit elektrischer Energie zumindest eine Batterieeinheit 6 auf, welche außerhalb des hermetisch abgedichteten Aufnahmeraums 3.3 vorgesehen ist, und zwar in zumindest einer Ausnehmung 3.4 des mehrteiligen Gehäuses 3, vorzugsweise in dessen unteren Gehäuseteil 3.2 vorgesehen ist. Eine beispielsweise zylinderförmig ausgebildete Batterieeinheit 6 ist in den Figuren 2, 3, 5 und 7 dargestellt.

Die Ausnehmung 3.4 ist vorzugsweise zur näherungsweise formschlüssigen Aufnahme der Batterieeinheit 6 ausgebildet. Beispielsweise weist die Ausnehmung 3.4 einen rechteckförmigen Querschnitt und/oder eine zumindest Teilzylinderform zur Aufnahme einer zylinderförmig ausgebildeten Batterieeinheit 6 auf.

Das mehrteilige Gehäuse3 weist ferner vorzugsweise im unteren Gehäuseteil 3.2 zumindest eine Batterieauswurfbohrung 3.5 auf, die mit der Ausnehmung 3.4 in Verbindung steht. Die Batterieauswurfbohrung 3.5 erstreckt sich hierbei vorzugsweise parallel zur Längsachse LA des Gehäuses 3 der Fernbedienung 1, und zwar unterhalb des den Aufnahmeraum 3.3 bildenden Abschnittes des unteren Gehäuseteil 3.2, und zwar schließt sich diese unmittelbar an die Ausnehmung 3.4 entlang der Längsachse LA an.

Die Ausnehmung 3.4 weist somit eine in einem ersten Gehäuserandabschnitt 3' vorgesehene Aufnahmeöffnung 3.4' und die Batterieauswurfbohrung 3.5 eine in einem zweiten Gehäuserandabschnitt 3" vorgesehene erste Bohrungsöffnung 3.5' auf, die einander gegenüberliegend angeordnet sind, wobei im Bodenbereich 3.4" der Ausnehmung 3.4 eine zweite Bohrungsöffnung 3.5" der Batterieauswurfbohrung 3.5 vorgesehen ist. Über die erste Bohrungsöffnung 3.5' kann somit ein längliches, stiftartiges Element in die Batterieauswurfbohrung 3.5 eingeführt werden, welches über die zweite Bohrungsöffnung 3.5" der Batterieauswurfbohrung 3.5 in den Aufnahmeraum 3.4 gelangt und somit ein Ausstoßen der Batterieeinheit 6 aus der Ausnehmung in Richtung der Längsachse LA bewirkt.

Ferner sind in der Ausnehmung 3.4 zumindest ein erstes und zweites Kontaktelement 7.1, 7.2 zum elektrischen Anschluss des Plus- und Minuspols der Batterieeinheit 6 vorgesehen, wobei vorzugsweise das erste Kontaktelement 7.1 vollständig in der Ausnehmung 3.4 aufgenommen ist und zweite Kontaktelement 7.2 zumindest teilweise über die Aufnahmeöffnung 3.4' nach außen weg steht. Das erste und zweite Kontaktelement 7.1, 7.2 sind hierbei einander gegenüberliegend angeordnet, und zwar jeweils parallel zur Längsachse LA des Gehäuses 3.

Das erste und zweite Kontaktelement 7.1, 7.2 sind ferner zur Aufnahme der stirnseitigen Enden der beispielsweise zylinderförmig ausgebildeten Batterieeinheit 6 eingerichtet. Hierbei weist zumindest eines der Kontaktelemente 7.1, 7.2, im vorliegenden Ausführungsbeispiel das zweite Kontaktelement 7.2, eine Kontaktfederzunge 7.2' zur Erzeugung einer Federkraft auf, über welche die Batterieeinheit 6 zwischen erstem und zweitem Kontaktelement 7.1, 7.2 eingeklemmt wird.

Das erste und zweite Kontaktelement 7.1, 7.2 sind über elektrische Verbindungselemente 8, 8' mit der zumindest einen Elektronikeinheit 4 verbunden, welche beispielsweise durch einen elektronischen Schaltkreis gebildet ist, der auf einer Leiterplatine zusammen mit dem zumindest einem Bedienelement 5 angeordnet ist.

Die Betätigung des auf der Leiterplatine montierten zumindest einen Bedienelementes 5 erfolgt hierbei über eine Betätigungsmechanik, die zumindest teilweise durch einen einstückig mit dem oberen Gehäuseteil 3.1 ausgebildeten Betätigungsabschnitt 3.11 gebildet ist, der entweder direkt oder indirekt mit dem zumindest einen Bedienelement 5 in Wirkverbindung steht. Hierzu ist das obere Gehäuseteil 3.1 zumindest im Betätigungsabschnitt 3.11 zumindest teilweise elastisch ausgebildet.

Im vorliegenden Ausführungsbeispiel weist die Fernbedienung 1 mehrere Bedienelemente 5, und zwar beispielsweise eine Steuerscheibe 5.1 und eine erste und zweite Funktionstaste 5.2, 5.3 auf, denen jeweils ein Betätigungsabschnitt 3.11, 3.12, 3.13 im obere Gehäuseteil 3.1 zugeordnet ist.

Die Betätigungsabschnitte 3.11, 3.12, 3.13 sind vorzugsweise kreisförmig ausgebildet und weisen einen konzentrisch um Ihre Mittelachse verlaufenden ringförmigen Scharnierabschnitt auf, welcher eine schalterartige Bewegung der Betätigungsabschnitte 3.11, 3.12, 3.13 des oberen Gehäuseteils 3.1 entlang der jeweiligen Mittelachse ermöglicht.

Die Steuerscheibe 5.1 ist zur Realisierung der von einem Joystick bereitgestellten Steuerfunktion ausgebildet. Mittels der Steuerscheibe 5.1 kann somit das die Spitze eines chirurgischen Instrumentes, beispielsweise die Optik einer Kameraeinheit in einem kartesischen Raumkoordinatensystem, und zwar in der X, Y-Ebene nahezu beliebig gesteuert bewegt werden. Über die erste und zweite Funktionstaste 5.2, 5.3 kann beispielsweise die Zoomfunktion einer derartigen Kameraeinheit, d.h. eine Steuerung entlang der Z-Achse, realisiert werden. Hierdurch werden die sechs Bewegungsrichtung +/- X, +/- Y, +/-Z innerhalb eines kartesischen Raumkoordinatensystems abgebildet.

In einer bevorzugten Ausführungsform umfasst die Betätigungsmechanik der Steuerscheibe 5.1 zumindest eine kreuzartige Steuerwippe 5.11, welche unterhalb des zugeordneten Betätigungsabschnittes 3.11 des oberen Gehäuses 3.1 angeordnet ist, und zwar kommt der Kreuzungspunkt der kreuzartigen Steuerwippe 5.11 im Schnittpunkt mit der Mittelachse des Betätigungsabschnittes 3.11 zu liegen.

Die kreuzartige Steuerwippe 5.11 bildet vorzugsweise vier Steuerarme aus, welche über einen im Kreuzungspunkt angelenkten Gelenkstift 5.12 beweglich gelagert sind, und zwar derart, dass jeweils zwei der Steuerarme ausgehend vom Kreuzungspunkt in Richtung der Längsachse LA und in Richtung einer senkrecht zur Längsachse LA orientierten Achse jeweils in entgegen gesetzter Richtung bewegbar sind. Der Gelenkstift 5.12 ist hierzu in seinem mit der kreuzartigen Steuerwippe 5.11 in Wirkverbindung stehenden freien Ende zumindest halbkugelartig ausgebildet.

Die freien Enden der vier Steuerarme der kreuzartigen Steuerwippe 5.11 stehen in Wirkverbindung mit jeweils einem elektrischen Schaltelement, dessen Betätigung über den elektronischen Schaltkreis der Elektronikeinheit 4 erfassbar ist und welche abhängig davon Steuerbefehle erzeugt, die über die drahtlose Schnittstelle an die Steuereinrichtung des Operations-Assistenz-Systems übertragen werden.
Die Erfindung wurde voranstehend an einem Ausführungsbeispiel beschrieben. Es versteht sich, dass zahlreiche Änderungen sowie Abwandlungen möglich sind, ohne das dadurch der der Erfindung zugrunde liegende Erfindungsgedanke verlassen wird.

Beispielsweise kann der Aufnahmeraum 3.3 nach Einsetzen der Elektronikeinheit 4 zumindest teilweise vergossen werden, und zwar vorzugsweise mit einem harzartigen Material.

Ferner ist eine Klemmvorrichtung 9 zur Befestigung der erfindungsgemäßen Fernbedienung 1 an einem chirurgischen Instrument 2 vorgesehen, welche beispielsweise zumindest eine Klemmschraube 9' umfasst.

### Bezugszeichenliste

- 1: Fernbedienung
- 2: chirurgisches Instrument
- 3: Mehrteiliges Gehäuse
- 3': erster Gehäuserandabschnitt
- 3": zweiter Gehäuserandabschnitt
- 3.1: oberes Gehäuseteil
- 3.11-3.13: Betätigungsabschnitte
- 3.2: unteres Gehäuseteil
- 3.3: Aufnahmeraum
- 3.4: Ausnehmung
- 3.4': Aufnahmeöffnung
- 3.4": Bodenbereich
- 3.5: Batterieauswurfbohrung
- 3.5': erste Bohrungsöffnung
- 3.5": zweite Bohrungsöffnung
- 4: Elektronikeinheit
- 5: Bedienelement
- 5.1: Steuerscheibe
- 5.11: kreuzartige Steuerwippe
- 5.12: Gelenkstift
- 5.2: erste Funktionstaste
- 5.3: zweite Funktionstaste
- 6: Batterieeinheit
- 7.1: erstes Kontaktelement
- 7.2: zweites Kontaktelement
- 7.2': Kontaktfederzunge
- 8, 8': Verbindungselemente
- 9: Klemmvorrichtung
- 9': Klemmvorrichtung

## Patentansprüche

1. Fernbedienung für Operations-Assistenz-Systeme bestehend aus einem mehrteiligen Gehäuse (3) zur Aufnahme zumindest einer Elektronikeinheit (4) und zumindest einem mit der Elektronikeinheit (4) verbundenen Bedienelementes (5), wobei das Gehäuse (3) zumindest ein oberes und unteres Gehäuseteil (3.1, 3.2) aufweist, die zur Ausbildung eines autoklavierbaren Gehäuses (3) hermetisch dicht miteinander verbunden sind, **dadurch gekennzeichnet, dass** der untere Gehäuseteil (3.2) zumindest eine Ausnehmung (3.4) zur Aufnahme einer Batterieeinheit (6) und zumindest eine Batterieauswurfbohrung (3.5) zum vereinfachten Entnehmen der Batterieeinheit (6) aus der Ausnehmung (3.4) aufweist, die mit der Ausnehmung (3.4) in Verbindung steht, und dass die Ausnehmung (3.4) eine in einem ersten Gehäuserandabschnitt (3') vorgesehene Aufnahmeöffnung (3.4') und die Batterieauswurfbohrung (3.5) eine in einem zweiten Gehäuserandabschnitt (3") vorgesehene erste Bohrungsöffnung (3.5') und im Bodenbereich (3.4") der Ausnehmung (3.4) eine zweite Bohrungsöffnung (3.5") aufweist, wobei die Aufnahmeöffnung (3.4') und die vorgesehene erste Bohrungsöffnung (3.5') einander gegenüberliegend angeordnet sind.

2. Fernbedienung nach Anspruch 1, **dadurch gekennzeichnet, dass** das obere und untere Gehäuseteil (3.1, 3.2) randseitig miteinander verklebt sind.

3. Fernbedienung nach Anspruch 1, **dadurch gekennzeichnet, dass** die Ausnehmung (3.4) zur näherungsweise formschlüssigen Aufnahme der Batterieeinheit (6) ausgebildet ist.

4. Fernbedienung nach einem der vorhergehenden Ansprüche, **dadurch gekennzeichnet, dass** die Fernbedienung (1) über eine drahtlose Schnittstelle mit einer Steuereinrichtung eines Operations-Assistenz-Systems verbindbar ist.

5. Fernbedienung nach einem der vorhergehenden Ansprüche, **gekennzeichnet durch** die Verwendung zur Steuerung eines chirurgischen Hilfsinstrumentes, insbesondere eines ein Endoskop aufweisendes Kamerasystems mittels eines Operations-Assistenz-Systems.

## Claims

1. Remote control for operation assistance systems consisting of a multiple-part housing (3) for receiving at least one electronic unit (4) and at least one operating element (5) connected to the electronic unit (4), wherein the housing (3) comprises at least one upper and lower housing part (3.1, 3.2) which are connected to each other in a hermetically sealed manner to form an autoclavable housing (3), **characterised in that** the lower housing part (3.2) comprises at least one recess (3.4) for accommodating a battery unit (6) and at least one battery ejection bore (3.5), for the simplified removal of the battery unit (6) from the recess (3.4), which is connected to the recess (3.4), and **in that** the recess (3.4) comprises a recess opening (3.4') provided in a first housing edge section (3') and the battery ejection bore (3.5) comprises a first bore opening (3.5') provided in a second housing edge section (3") and a second bore opening (3.5") in the base area (3.4") of the recess (3.4), wherein the recess opening (3.4') and the provided first boring opening (3.5') are arranged opposite each other.

2. Remote control according to claim 1 **characterised in that** the upper and lower housing parts (3.1, 3.2) are adhesively joined to each other at the edge.

3. Remote control according to claim 1 **characterised in that** the recess (3.4) is designed for the approximately positively locked accommodation of the battery unit (6).

4. Remote control according to any one of the preceding claims **characterised in that** the remote control (1) can be connected via a wireless interface to a control means of an operation assistance system.

5. Remote control according to any one of the preceding claims **characterised by** its use for controlling a surgical auxiliary instrument, more particularly a camera system having an endoscope, by means of an operation assistance system.

## Revendications

1. Télécommande pour des systèmes d'assistance à l'opération composée d'un boîtier en plusieurs parties (3) pour recevoir au moins une unité électronique (4) et au moins un élément de commande (5) relié à l'unité électronique (4), le boîtier (3) comportant au moins une partie de boîtier supérieure et une partie de boîtier inférieure (3.1, 3.2), qui sont reliées l'une à l'autre de façon hermétiquement étanche pour constituer un boîtier (3) pouvant passer à l'autoclave, **caractérisée en ce que** la partie de boîtier inférieure (3.2) comporte au moins un évidement (3.4) pour recevoir une unité de batterie (6) et au moins un trou d'éjection de batterie (3.5) pour simplifier l'enlèvement de l'unité de batterie (6) de l'évidement (3.4), qui est en liaison avec l'évidement (3.4) et **en ce que** l'évidement (3.4) comporte une ouverture de réception (3.4') prévue dans une première section de bordure de boîtier (3') et le trou d'éjection de batterie (3.5) comporte une première ouverture de trou (3.5') prévue dans une deuxième section de bordure de boîtier (3") et une deuxième ouverture de trou (3.5") dans la zone de fond (3.4") de l'évidement (3.4), dans lequel l'ouverture de réception (3.4') et la première ouverture de trou (3.5') prévue sont disposées opposées l'une à l'autre.

2. Télécommande selon la revendication 1, **caractérisée en ce que** la partie de boîtier supérieure et la partie de boîtier inférieure (3.1, 3.2) sont collées l'une à l'autre sur les bords.

3. Télécommande selon la revendication 1, **caractérisée en ce que** l'évidement (3.4) est constitué pour la réception approximativement par conformité de forme de l'unité de batterie (6).

4. Télécommande selon l'une quelconque des revendications précédentes, **caractérisée en ce que** la télécommande (1) peut être reliée à un dispositif de commande d'un système d'assistance à l'opération par le biais d'une interface sans fil.

5. Télécommande selon l'une quelconque des revendications précédentes, **caractérisée par** l'utilisation pour la commande d'un instrument d'assistance chirurgical, notamment d'un système de caméra comportant un endoscope, au moyen d'un système d'assistance à l'opération.
